# EUROPEAN PATENT SPECIFICATION

(11) **EP 2 525 211 B1**
(45) Date of publication and mention of the grant of the patent: **03.01.2018**
(21) Application number: 11166215.1
(22) Date of filing: 16.05.2011
(51) Int. Cl.: G01N 21/64

(54) **INSTRUMENT AND METHOD FOR DETECTING ANALYTES**
INSTRUMENT UND VERFAHREN ZUM DETEKTIEREN VON ANALYTEN
INSTRUMENT ET PROCÉDÉ DE DÉTECTION D'ANALYTES

(43) Date of publication of application: 21.11.2012
(73) Proprietor: F. Hoffmann-La Roche AG, 4070 Basel (CH); Roche Diagnostics GmbH, 68305 Mannheim (DE)
(72) Inventor: Furlan, Alan, 6300, Zug (CH)
(74) Representative: Peterreins Schley

(56) References cited:
- EP-A1- 1 677 097
- EP-A1- 1 681 555
- WO-A1-02/48690
- US-A1- 2003 011 772
- US-A1- 2004 009 586

## Description

### TECHNICAL FIELD

The present invention is in the field of biochemical research, biochemical routine analytics, clinical diagnostics and clinical research and more particularly concerns an instrument and method for the automated detection of analytes by measuring the emission of light of two or more luminescence labels indicating the presence of analytes. It further pertains to a system for analyzing sample fluids comprising an instrument for detecting analytes.

### BACKGROUND OF THE INVENTION

In these days, there are various methods of detecting the presence of analytes in sample fluids. It is common practice to mark analytes by fluorescence dyes enabling an optical detection by detecting light emitted from the dyes indicating the presence, amount or concentration in response to excitation light. These labels do not necessarily need to be chemically bound to the analyte; it is also very common to use labels that emit light once they are freed from a molecule they were originally bound to. The fluorescence signal obtained from these dyes usually correlates to the amount of analyte present in the sample fluid.

Specifically, in the case of using the well-known polymerase chain reaction (PCR) to replicate target nucleic acids to an amount which is better detectable, the nucleic acids are combined with reagents containing fluorescence dyes which can react with the nucleic acids so as to obtain dye-marked nucleic acids. The dye-marked nucleic acids are repeatedly put through a sequence of amplification steps, wherein each sequence includes steps of melting the double-stranded nucleic acids to obtain denaturated single polynucleotide strands, annealing short primers to the strands and extending those primers to synthesize new polynucleotide strands along the denaturated strands to make new copies of double-stranded nucleic acids. Due to the fact that reaction conditions strongly vary with temperature, the samples are put through a series of temperature excursions in which predetermined temperatures are kept constant for specific time intervals ("thermo-cycling"). The reaction products are optically detected, e.g., during the progress of the PCR (real-time PCR), by exciting the dyes indicating the presence of the nucleic acids and measuring the fluorescence signal emitted in response to the excitation light. The fluorescence signal obtained correlates to the amount of nucleic acids. In daily routine, commercially available instruments are used for performing the PCR and optical detection of the reaction products.

The international patent application WO 02/48690 relates to an instrument for measuring fluorescence light comprising two filter wheels in an excitation and emission beam path. In table 2, two dyes are cited, one of which having an excitation wavelength corresponding to the emission wavelength of the other dye.

In light of the foregoing, it is an object of the invention to provide an automated low-cost instrument and method for the precise optical detection of analytes such as reaction products of the PCR. These and further objects are met by an instrument and method according to the independent claims and as described herein below. Preferred embodiments of the invention are given by the features of the dependent claims and as described herein below.

### SUMMARY OF THE INVENTION

According to a first aspect of the invention, a new instrument for the automated detection of at least one analyte in a sample is proposed. The instrument can be used to detect analytes by measuring light emitted by two or more luminescence labels indicating the analytes in response to excitation light. Stated more particularly, the instrument can, e.g., be used to detect at least two different analytes wherein each analyte is being indicated by one label, the labels of the various analytes being different with respect to each other. Otherwise, the instrument can, e.g., be used to detect one or more analytes, each of which being indicated by two or more labels which are different with respect to each other. By detecting the light emitted by the analyte-indicating labels, the presence and optionally amount or concentration of the analytes can be determined. The luminescence labels can, e.g., correspond to the analytes by reacting the analytes with one or more reagents containing the labels. Luminescence labels for use in the present invention can, e.g., be chosen from rhodamine, courmarine, cyanine dyes and derivates thereof. The luminescence labels can, e.g., be chosen as fluorescence dyes, wherein the specific value of the fluorescence signal obtained usually correlates to the amount of analyte contained in the sample.

In general, the instrument of the invention will especially be useful for detecting the light emitted by two or more labels different with respect to each other by having different excitation and emission peaks. As used herein, the term "peak" refers to the wavelength or range of wavelengths causing a maximum intensity of light emitted by the related label. The labels are chosen in a manner that the excitation peak of one label at least approximately corresponds to the emission peak of another label.

As used herein, the term "analyte" refers to any substance the presence and optionally amount or concentration of which can be determined by measuring the emission of light of at least one luminescence label related thereto.

As used herein, the term "sample" refers to any substance in which the analyte can be contained. Samples can, e.g., be liquid samples or dry samples. For example, analytes can be contained in chemical fluids which can be subject to one or more chemical analyses and assays, e.g., drug interaction screening, environmental analysis, identification of organic substances, etc. Analytes can, e.g., be contained in biological fluids such as body fluids, e.g., blood, serum, urine, saliva, cerebrospinal fluid etc. which can be subject to one or more analyses and assays in medical and pharmaceutical research and clinical diagnosis which may involve in-vitro amplification techniques, e.g., based on the polymerase chain reaction (PCR) or any other reaction of the nucleic acid amplification type. Analytes can, e.g., be contained in pre-processed body fluids such as extracts of body fluids containing target nucleic acids used as starting materials, e.g., for the PCR. Analytes can also be contained in any other fluid of interest. For example, analytes can be nucleic acids or segments thereof, proteins, antibodies, cells and many others.

The instrument of the invention will particularly be useful for the optical (e.g. on-line) detection of PCR reaction products. Specifically, the instrument can, e.g., be used for optically detecting the products of the PCR with hybridization probes, PCR with hydrolysis probes, PCR with interchelator dyes, real-time PCR with corresponding probes, various isothermal amplification methods with corresponding fluorescence reporters and melting analysis of DNA. Typical optical analyses are the detection of the presence/absence and optionally concentration of pathogens such as virus or bacteria in a sample, genotyping, measuring expression profiles, and many others.

According to the invention, as an excitation component, the instrument comprises at least one light source, capable of generating light for exciting two or more luminescence labels different with respect to each other. The at least one light source can, e.g., be chosen from a white light source such as, but not limited to, a halogen lamp and a white light emitting diode (LED), or from two or more quasi-monochromatic (multi-coloured) LEDs. The instrument further comprises an excitation beam path extending between the at least one light source and the sample (analyte) for propagation of the light generated by the at least one light source.

As a detection component, the instrument of the invention further comprises at least one detector capable of detecting light emitted from the sample, e.g., in response to excitation by the excitation light. The at least one detector can, e.g., be configured as charge coupled device (CCD), CMOS detector, camera, photomultiplier, photodiodes and many others. The instrument further comprises an emission beam path extending between the sample (analyte) and the detector for propagation of the light emitted from the sample.
As a light filtering component, the instrument of the invention further comprises one filter carrier carrying more than two pairs of filter portions, wherein each pair of filter portions is related to an individual luminescence label. Stated more particularly, each pair of filter portions is related to one luminescence label wherein different pairs of filter portions are related to different luminescence labels. Each pair of filter portions comprises one filter portion (in the following denoted as "first filter portion"), adapted to transmit excitation light generated by the at least one light source for exciting the related luminescence label and another filter portion (in the following denoted as "second filter portion"), adapted to transmit light emitted by the related label, wherein the first filter portion of one pair is the second filter portion of another pair. Furthermore, the filter portions are arranged in a manner that a respective one of the pairs of filter portions can be brought in a condition (in the following denoted as "operative condition") in which the first filter portion is arranged in the excitation beam path and the second filter portion is arranged in the emission beam path. In other words, the filter portions are arranged in a manner that one pair of filter portions can selectively be brought in operative condition while all other pairs of filter portions are not in operative condition, i.e. in a condition in which at least one filter portion thereof is not arranged in the excitation or emission beam paths (in the following denoted as "non-operative condition.") . Otherwise, each one of the pairs of filter portions can be brought in operative condition.
In order to bring individual pairs of filter portions in operative condition, the filter carrier and the beam paths are movable with respect to each other by means of at least one moving mechanism. Stated more particularly, in the instrument of the invention, the moving mechanism is embodied in a manner that the filter carrier is movable with respect to the stationary beam paths. Specifically, according to one embodiment of the invention, the moving mechanism is adapted to rotate the filter carrier in distinct positions with respect to the stationary beam paths so as to rotate a respective one of the pairs of first and second filter portions in operative condition by equal rotation steps. According to one alternative embodiment, the moving mechanism is adapted to translate the filter carrier in distinct positions with respect to the stationary beam paths so as to translate a respective one of the pairs of first and second filter portions in operative condition by equal translation steps. In these embodiments, it is preferred that the instrument comprises one carrier drive for moving the filter carrier in distinct positions relative to the excitation and emission beam paths. The carrier drive may be connected to a controller, set up to control the activity of the carrier drive.
Otherwise, according to one alternative embodiment which is not part of the invention, the moving mechanism is adapted to move the excitation and emission beam paths in distinct positions with respect to the stationary filter carrier so as to bring a respective one of the pairs of first and second filter portions in operative condition. This can, e.g., be realized by moving and/or changing the direction of light guiding and/or light shaping and/or light directing elements such as light fibers, mirrors, prisms and the like.
Accordingly, the instrument of the invention uses only one (common) filter carrier for carrying the first and second filter portions which, e.g., can be moved by one carrier drive so that costs in producing the instrument can advantageously be saved. Otherwise, the first filter portions of one pair of filter portions can be used as second filter in another pair of filter portions. Hence, by double-using filter portions, costs in fabricating the instrument can advantageously be saved. Otherwise, different pairs of filter portions sharing one filter portion can very quickly be brought in operative condition, by moving the filter carrier by equal moving steps facilitating the moving mechanism.

As above-detailed, the samples containing the analytes can, e.g., be liquid or dry samples. According to an embodiment related to liquid samples, the instrument can, e.g., comprise at least one mount for accommodating a sample carrier such as, but not limited to, a microplate comprising one or more wells, typically an array of wells, for containing liquid samples. According to an alternative embodiment related to dry samples, the instrument can, e.g., comprise at least one mount for accommodating a substrate with a surface comprising one or more analytes, e.g. covalently, attached to the surface in discrete regions, such as, but not limited to, groups of oligonucleotides having similar or different nucleotide sequences.
In the instrument of the invention, the filter carrier is carrying a plurality of filter portions. According to one embodiment, the filter carrier is provided with a plurality of distinct filter portions. For that purpose, the filter carrier may include a plurality of seats for (e.g. removably) fixing the filter portions. Specifically, the first and second filter portions of one pair of filter portions can, e.g., be separated by at least one seat that can be a blank seat (i.e. without filter portion) or can be provided with a filter portion of another pair of filter portions. Hence, neighbouring filter portions can belong to different pairs of filter portions.
According to an alternative embodiment, the filter carrier is carrying a one-piece filter comprised of the first and second filter portions, wherein the one-piece filter can have a continuous or discontinuous, i.e. discrete, transmission spectrum. Specifically, the one-piece filter may, e.g., include a plurality of distinct filter portions serially arranged with respect to each other.

According to a preferred embodiment, the filter carrier is provided with at least one opaque region, adapted to inhibit the transmission of light emitted by the sample that is arranged in a manner to be brought in the emission beam path, e.g., by moving the filter carrier. The opaque region can be, e.g., be used to calibrate the instrument.

According to a second aspect, a new method for detecting the presence and optionally amount or concentration of at least one analyte in a sample or on a pad is proposed. The method comprises a step of generating excitation light propagating along an excitation beam path towards said sample. It comprises a further step of detecting light emitted from the sample in response to the excitation light, propagating along an emission beam path. It comprises a further step of moving one (first) pair of first and second filter portions related to one luminescence label and said beam paths with respect to each other so that the (first) pair is in an operative condition in which the first filter portion thereof, adapted to transmit excitation light for exciting the related luminescence label, is arranged in the excitation beam path and the second filter portion thereof, adapted to transmit light emitted by the related luminescence label, is arranged in the emission beam path. It comprises a further step of moving another (second) pair of first and second filter portions related to another luminescence label and the beam paths with respect to each other so that the another (second) pair of filter portions is in the operative condition, wherein the first filter portion of the one (first) pair of filter portions is the second filter portion of the another (second) pair of filter portions.

According to a preferred embodiment, a filter carrier carrying the first and second filter portions is rotated in distinct rotating positions relative to the excitation and emission beam paths kept stationary so as to move a respective one of the pairs of filter portions in the operative condition. Alternatively, the filter carrier carrying the first and second filter portions is translated in distinct translating positions relative to the excitation and emission beam paths kept stationary so as to translate a respective one of the pairs of filter portions in the operative condition. According to the invention, the filter carrier is moved by equal moving, i.e., rotating and translating, respectively, steps.

According to an alternative embodiment, which is not part of the invention, the excitation and emission beam paths are moved in distinct positions with respect to the first and second filter portions kept stationary so as to bring a respective one of the pairs of first and second filter portions in operative condition.

According to a third aspect, a new system for analyzing a sample by detecting the presence and optionally amount or concentration of at least one analyte is proposed. The system comprises an instrument for detecting the presence of at least one analyte in a sample as above-detailed. Stated more particularly, the system comprises a temperature-controlled block for heating the sample, e.g., for thermo-cycling the sample. The system further comprises at least one light source capable of generating light for exciting two or more luminescence labels and an excitation beam path extending between the light source and the sample for propagation of the excitation light. The system further comprises at least one detector capable of detecting light emitted from the sample, e.g., in response to the excitation light, and an emission beam path extending between the sample and the detector for propagation of the emitted light. It further comprises one filter carrier carrying two or more pairs of filter portions, wherein each pair is related to one luminescence label comprising a first filter portion, adapted to transmit excitation light for exciting the related luminescence label, and a second filter portion, adapted to transmit light emitted by the related luminescence label, wherein the first filter portion of one pair is the second filter portion of another pair. In the system, the filter carrier is movable with respect to the excitation and emission beam paths by at least one moving mechanism so as to bring a respective one of the pairs of filter portions in an operative condition in which the first filter portion is in the excitation beam path and the second filter portion is in the emission beam path. The system further comprises one carrier drive for moving the filter carrier in distinct positions relative to the excitation and emission beam paths, and a controller set up to control activity of the carrier drive in a manner to move a respective one of the pairs of first and second filter portions in operative condition.

The above-described embodiments of the various aspects of the invention may be used alone or in any combination thereof without departing from the scope of the invention.

### BRIEF DESCRIPTION OF THE DRAWINGS

Other and further objects, features and advantages of the invention will appear more fully from the following description. The accompanying drawings, which are incorporated in and constitute a part of the specification, illustrate preferred embodiments of the invention, and together with the general description given above and the detailed description given below, serve to explain the principles of the invention. In the drawings, similar structures are referred to by like numerals throughout the various embodiments.
- FIG. 1: is a schematic drawing illustrating an embodiment of the instrument of the invention;
- FIGS. 2A-2C: are perspective drawings illustrating a rotatable filter arrangement of the instrument of FIG. 1;
- FIG. 3: is a schematic drawing illustrating various discrete filter portions of the rotatable filter arrangement of FIGS. 2A-2C;
- FIGS. 4A-4B: are schematic drawings illustrating an exemplary method using the rotatable filter arrangement of FIGS. 2A-2C;
- FIG. 5: is a schematic drawing illustrating a translatory filter arrangement of the instrument of FIG. 1 configured as two continuous filter strips;
- FIG. 6: is a schematic drawing illustrating another translatory filter arrangement of the instrument of FIG. 1 configured as one continuous filter strip;
- FIG. 7: is a schematic drawing illustrating various filter portions of the filter strips of FIGS. 5 and 6;
- FIG. 8: is a schematic illustration of various modules of an exemplary system for the automated thermal treatment of samples comprising an instrument as depicted in FIGS. 1 to 7.

### DETAILED DESCRIPTION OF THE INVENTION

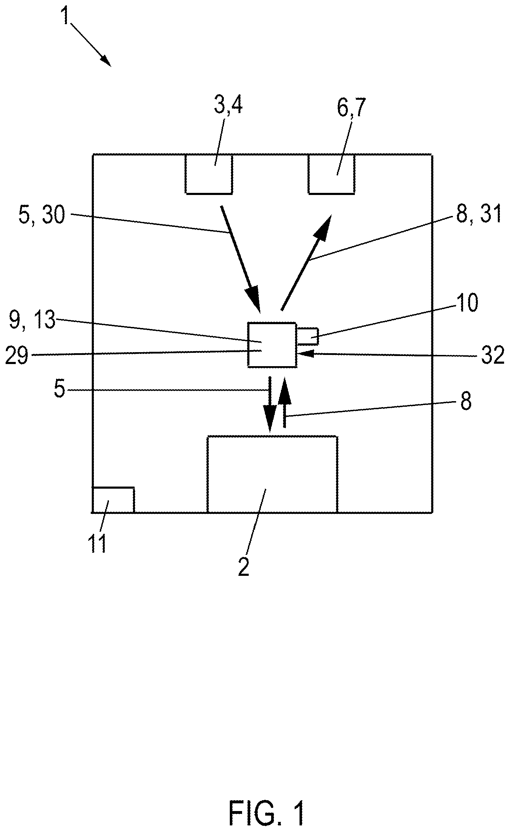

The present invention will be described in detail below with reference to the accompanying drawings. With particular reference to FIG. 1, an instrument, in general referred to at reference numeral 1, for detecting the presence and optionally amount or concentration of at least one analyte is explained. The instrument 1 can, e.g., be used to optically detect reaction products obtained by thermally cycling liquid reaction mixtures of nucleic acids and one or more reagents through a series of temperature excursions. Specifically, the instrument 1 can be used to detect reaction products of the PCR, in particular real-time PCR or any other reaction of the nucleic acid amplification type. The instrument 1 can in particular be used for the optical on-line detection of reaction products.
With continued reference to FIG. 1, the instrument 1 includes various components as detailed in the following description which are functional and (optionally) structural entities for detecting the presence/absence and optionally amount or concentration of at least one analyte. Specifically, as illustrated in FIG. 1, the instrument 1 includes an analyte region 2 for containing the one or more analytes the presence or absence of which is to be optically detected by means of at least two luminescence labels.
The instrument 1 further includes an excitation arrangement 3 provided with at least one light source 4 capable of generating light adapted for exciting at least two luminescence labels contained in the analyte region 2. The light source 4 can, e.g., be configured as a white light source such as a halogen lamp or a white light emitting diode (LED). Otherwise, multiple coloured LEDs having different wavelengths or ranges of wavelengths can be used. The purpose of the excitation arrangement 3 is to generate excitation light 30 for exciting two or more luminescence labels contained in the analyte region 2. The excitation light 30 propagates along an excitation beam path 5 extending between the excitation arrangement 3 and the analyte region 2 passing through a filter arrangement 9 provided with more than two pairs of filter portions as described in more detail below.

Specifically, an excitation optics (not further detailed in FIG. 1) can be used to transmit the excitation light 30 to the analyte region 2 via the excitation beam path 5.

With yet continued reference to FIG. 1, the instrument 1 includes a detection arrangement 6 provided with at least one detector 7 capable of detecting light 31 emitted from the analyte region 2, e.g., in response to the excitation light 30. Specifically, the detector 7 can be provided with one or more light-sensitive elements for optically detecting the light 31 emitted from the analyte region 2. The detector 7 can, e.g., be embodied as a lateral-resolving detector like a charge coupled device (CCDs) and a CMOS detector, a linear-array detector movable for scanning and a two-dimensional-array sensor such as a camera. In the instrument 1, light 31 emitted from the analyte region 2 propagates along an emission beam path 8 extending between the analyte region 2 and the detection arrangement 6 passing through the filter arrangement 9. Specifically, an emission optics (not further detailed in FIG. 1) can be used to transmit light emitted from the analyte region 2 via the emission beam path 8 to the detector 7.

The excitation and/or emission beam paths 5, 8 can include one or more light guiding and/or light shaping and/or light directing elements (not illustrated) such as, but not limited to, lenses and planar or bent mirrors and/or one or more light separating elements such as, but not limited to, transmission gratings, reflective gratings and prisms in order to transmit the excitation light 30 from the excitation arrangement 3 to the analyte region 2 and to detect light 31 emitted by the analyte region 2 by the detection arrangement 6.

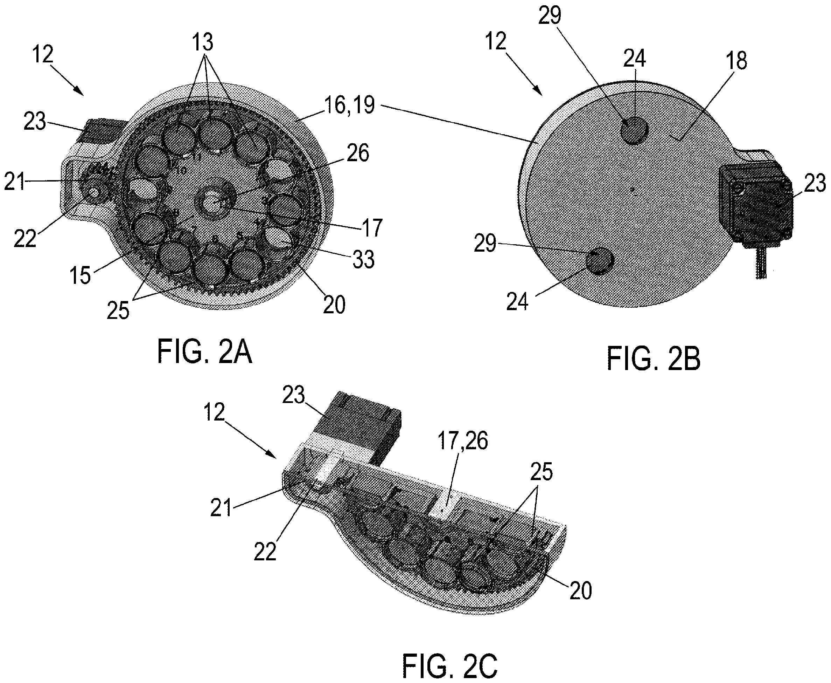

With yet continued reference to FIG. 1, the filter arrangement 9 is provided with more than two pairs 29 of filter portions 13. The filter arrangement 9 is movable with respect to the beam paths 5, 8 by means of a moving mechanism 32 not further detailed in FIG. 1. The moving mechanism 32 is coupled to at least one drive 10 for moving the filter arrangement 9 into distinct positions relative to the excitation and emission beam paths 5, 8.
With yet continued reference to FIG. 1, the instrument 1 further includes a controller 11 set up to control the activity of the various components of the instrument 1. The controller 11 can, e.g., be configured as programmable logic controller running a machine-readable program provided with instructions to perform operations for detecting the presence/absence and optionally amount or concentration of one or more analytes contained in the analyte region 2. Specifically, the controller 11 receives information from and generates and transmits control signals to the components which require control such as the drive 10, the at least one light source 4 and the at least one detector 7. Electric lines (not shown) can be used for transmitting the signals.
With particular reference to FIGS. 2A-2C, the filter arrangement 9 can, e.g., be embodied as a filter unit 12 illustrated by a front perspective view (FIG. 2A), a back perspective view (FIG. 2B) and a sectional perspective view thereof. Stated more particularly, the filter unit 12 includes a plurality of distinct first and second filter portions 13 fixed to a rotatable carrier or filter wheel 15. The filter wheel 15 is rotatably mounted to a wheel hub 17 protruding from a disk-like base portion 18 of a wheel casing 16. Specifically, the wheel casing 16 is comprised of the base portion 18 and a rim portion 19 surrounding the base portion 18 so as to form a trough accommodating the filter wheel 15. The filter wheel 15 is provided with an outer toothing 20 which is in meshing engagement with a pinion 21. The pinion 21 is driven by a shaft 22 of an electric motor 23 fixed to the base portion 18 of the wheel casing 16. Accordingly, the filter wheel 15 can be rotated around the central wheel hub 17 thereby defining a spin axis 26 so as to rotate the filter portions 13.

As illustrated in FIG. 2B, the base portion 18 of the wheel casing 16 is provided with two circular openings 24, i.e. a first opening 24 and a second opening 24, which in radial direction have an equal radial distance from the central wheel hub 17 or spin axis 26. Otherwise, in circumferential direction the openings 24 are arranged in a manner that the radii passing through the openings 24 enclose an angle of 150°. Specifically, in the instrument 1, the first opening 24 is located in the excitation beam path 5 and the second opening 24 is located in the emission beam path 8.

As illustrated in FIGS. 2A and 2C, the filter wheel 15 is provided with a plurality of circular seats 25 the diameter of which corresponds to the diameter of the circular openings 24. While a number of twelve seats 25 is shown for the purpose of illustration only, those of skill in the art will appreciate that any other number of seats 25 can be envisaged according to the specific demands of the user. As illustrated, the seats 25 are circumferentially arranged with respect to each other so as to have an equal radial distance with respect to the central wheel hub 17 or spin axis. Otherwise, the radii passing through two adjacent seats 25 enclose an angle of 30°. Each seat 25 can be loaded with one filter portion 13 wherein the seats 25 are adapted for removably fixing the filter portions 13 by means of a fixation mechanism such as, but not limited to, a catch mechanism, clamp mechanism or the like. Due to the specific arrangement of the seats 25 and the openings 24 two seats 25 the radii of which enclose an angle of 150° can be brought in a position to simultaneously overlap the openings 24. Hence, a first filter portion 13 and a second filter portion 13, that is to say, a pair 29 of first and second filter portions 13 can be positioned in the excitation and emission beam paths 5, 8 while each other filter portion 13 is outside the excitation and emission beam paths 5, 8.

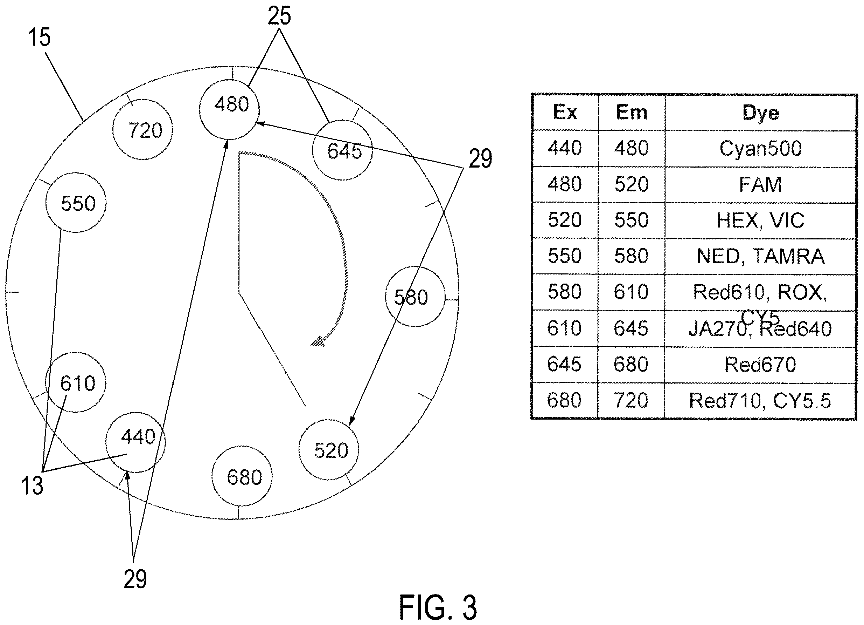

With particular reference to FIG. 3, an exemplary loading of the filter wheel 15 with various discrete filter portions 13 is illustrated. As illustrated, the filter portions 13 relate to a number of eight fluorescence labels or dyes as indicated in the table of FIG. 3. These dyes differ both in their excitation peaks and emission peaks as indicated by the wavelength (nm) for a maximum intensity (central wavelength) of the excitation spectrum (Ex) and emission spectrum (Em) . These dyes are selected in a manner that the Stokes shift of the dyes is rather similar with respect to each other, wherein the emission peak of one dye at least approximately corresponds to the excitation peak of another dye. For example, the dye "Cyan 500" has an emission peak of 480 nm corresponding to the excitation peak of the dye "FAM". Otherwise, the dye "FAM" has an emission peak of 520 nm corresponding to the excitation peak of the dyes "HEX, VIC" and so on.

As illustrated, the filter wheel 15 is loaded with filter portions 13 in a manner that one pair 29 of first and second filter portions 13 the radii of which enclose an angle of 150° relate to one dye. In other words, for each dye the filter wheel 15 includes one pair 29 of first and second filter portions 13 the radii of which enclose an angle of 150° wherein the first filter portion 13 is adapted to transmit the excitation light and the second filter portion 13 is adapted to transmit light emitted by the dye in response to the excitation light. Accordingly, the filter wheel 15 includes a number of eight pairs 29 of first and second filter portions 13 each of which being related to an individual dye, wherein each first filter portions 13 of one pair 29 corresponds to the second filter portion 13 of another pair 29 or vice versa so as to double-use one filter portion 13 for both pairs 29.

In the following, for the ease of explanation only, the position of the various pairs 29 of filter portions 13 of the filter wheel 15 is denoted by referring to a clock's notation as indicated in FIG. 2A. As illustrated, a pair 29 of first and second filter portions 13 of "Cyan 500" is located at 7 and 12 o'clock, a pair 29 of first and second filter portions 13 of "FAM" is located at 12 and 5 o'clock, a pair 29 of first and second filter portions 13 of "HEX, VIC" is located at 5 and 10 o'clock, a pair 29 of first and second filter portions 13 of "NED, TAMRA" is located at 10 and 3 o'clock, a pair 29 of first and second filter portions 13 of "RED610, ROX" is located at 3 and 8 o'clock, a pair 29 of first and second filter portions 13 of "JA270, CY5, Red640" is located at 8 and 1 o'clock, a pair 29 of first and second filter portions 13 of "Red670" is located at 1 and 6 o'clock, and a pair 29 of first and second filter portions 13 of "Red710, CY5.5" is located at 6 and 11 o'clock.

Accordingly, individual pairs 29 of first and second filter portions 13 have radii enclosing an angle of 150° and thus can selectively be brought in overlap with the openings 24 by rotating the filter wheel 15 by 150° or multiples thereof. Each of the filter portions 13 can be used for the filtering the excitation light of one dye and the light emitted from another dye. As a result, eight dyes can be excited and light emitted therefrom can be detected by turning the filter wheel 15 eight times by 150° (i.e. eight one-step turns) . Accordingly, cost for the production of the filter unit 12 and time for detecting the presence of analytes involving the use of two or more dyes can advantageously be saved. While a number of eight pairs 29 related to eight dyes of filter portions 13 is shown for the purpose of illustration only, those of skill in the art will appreciate that any other number of dyes and pairs 29 of filter portions 13 can be envisaged according to the specific demands of the user. While adjacent seats 25 are arranged to enclose an angle of 30°, those of skill in the art will appreciate that any other angle can be envisaged according to the specific demands of the user wherein the angle between the openings 24 has to be adapted correspondingly. Since the first and second filter portions 13 of one pair 29 of filter portions are separated by three seats 25 which are either blank seats or provided with filter portions 13 belonging to other pairs 29 of filter portions, the exciting and emitted light is spatially separated from each other so as to effectively avoid crosstalk.

As illustrated in FIG. 2A, in the filter unit 12, the filter wheel 15 can be open at the seats 25 so that light can pass through the seats 25 in case filter portions 13 are in overlap with the base portion openings 24. Otherwise, at 2, 4, and 9 o'clock there are blank seats 25. Alternatively, blank seats 25 can be closed by a cover (not illustrated) so as to block light to pass therethrough to thereby form opaque regions 33 which can, e.g., be used to calibrate the instrument 1.

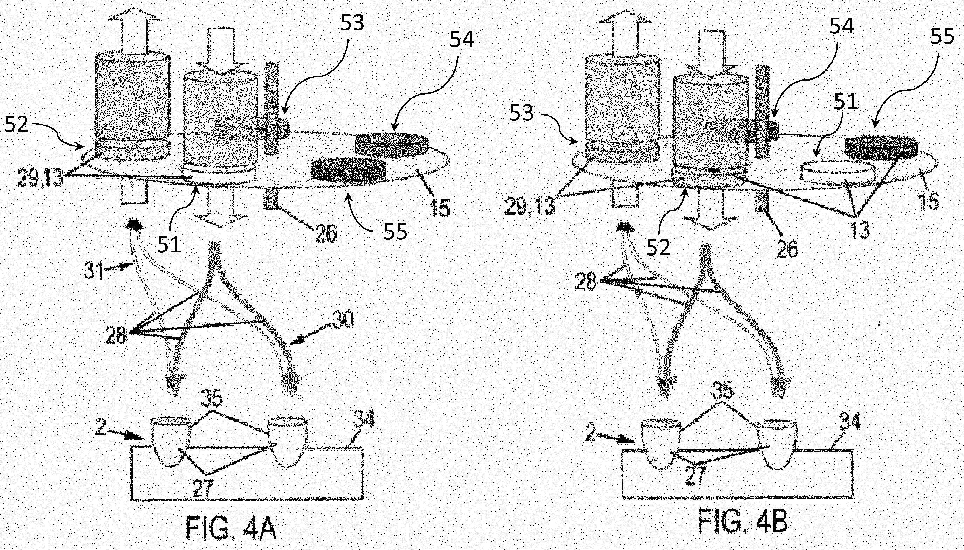

With particular reference to FIGS. 4A-4B an exemplary method for detecting the presence and optionally amount or concentration of at least one analyte by measuring the emission of light of dyes using the instrument 1 provided with the filter wheel 15 of FIGS. 2 to 3 is explained. In FIGS. 4A-4B, for the purpose of ease of illustration only, the filter wheel 15 is shown to be provided with a number of five filter portions 13. Two samples 27 containing the analyte are located in the analyte region 2 placed on a mount 34 supporting sample vessels 35 containing the samples 27. Both excitation and emission fibers 28 are used for guiding light 30, 31 towards or away from the samples 27.

FIG. 4A illustrates a first rotating position of the filter wheel 15 in which one pair 29 of filter portions 13 related to one dye, e.g. Cyan 500, is in operative position in which the first and second filter portions 13 are in overlap with the openings 24. Accordingly, the first filter portion 13, adapted to transmit and filter excitation light 30 having an excitation peak of 440 nm, is located in the excitation beam path 5 so that the dye can be excited by light 30 directed towards the samples 27 via the excitation fibers 28. Otherwise, the second filter portion 13, adapted to transmit and filter light 31 emitted by the samples 27 in response to the excitation light 30 having an emission peak of 480 nm, is located in the emission beam path 8 so that the light 31 collected by the emissions fibers 28 can be detected by the detector 7. The output of the emission fibers 28, e.g., is imaged onto a camera chip and detected with spatial resolution. The central wavelength of the second filter portion 13 is by about 40 nm longer than that one of the first filter portion 13 corresponding to a typical Stokes shift of fluorescent dyes.

Now turning the filter wheel 15 into a second rotating position, as illustrated in FIG. 4B, another pair 29 of filter portions 13 related to another dye different from the former one, e.g. FAM, is in operative position, wherein the second filter portion 13 of the former dye is the first filter portion 13 of the present dye or vice versa. Accordingly, the first filter portion 13 adapted to transmit and filter light 30 having an excitation peak of 480 nm is located in the excitation beam path 5 so that the dye can be excited. Otherwise, the second filter portion 13 adapted to transmit and filter light 31 emitted by the samples 27 in response to the excitation light having an emission peak of 520 nm is located in the emission beam path 8 so that emitted light can be detected by the detector 7. Accordingly, light 31 emitted by two dyes can readily be detected by the detector 7 involving a single or one-step turn of the filter wheel 15. Accordingly, by rotating the filter wheel 15 by a number of one-step turns, a same number of dyes can be excited and light emitted therefrom can be detected in response to the excitation light.

Furthermore, the filter wheel 15 can be brought in a rotating position in which blank seats 25 provide forming an opaque region 33 are in overlap with the openings 24 so as to inhibit light transmission towards the analyte region 2 and/or towards the detector 7 (by a cover on the blank seats 25 or providing for closed blank seats 25 as above-described). Accordingly, the detector 7 can be tested for the detection of light not originating from the analyte region 2 so as to improve the reliability of the detection results.

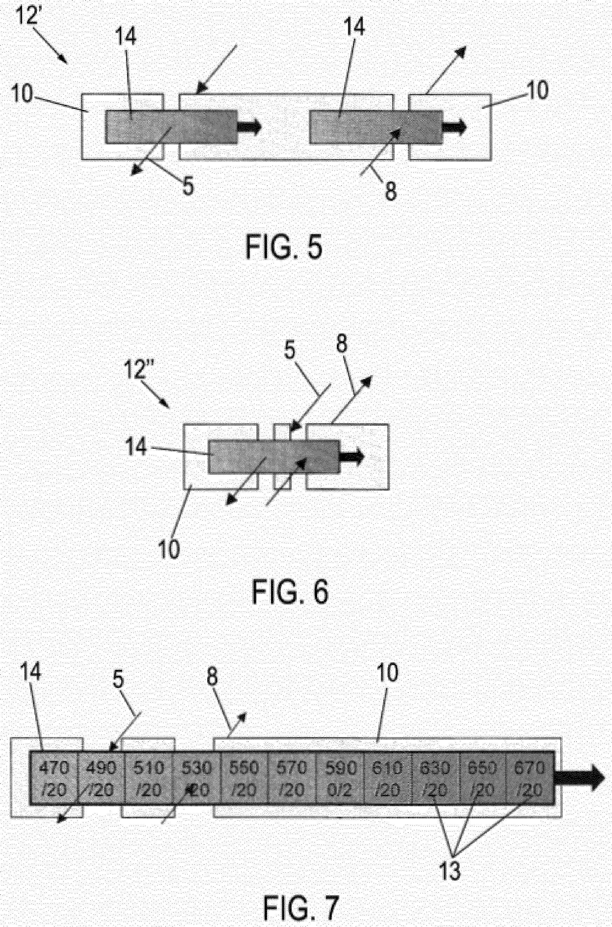

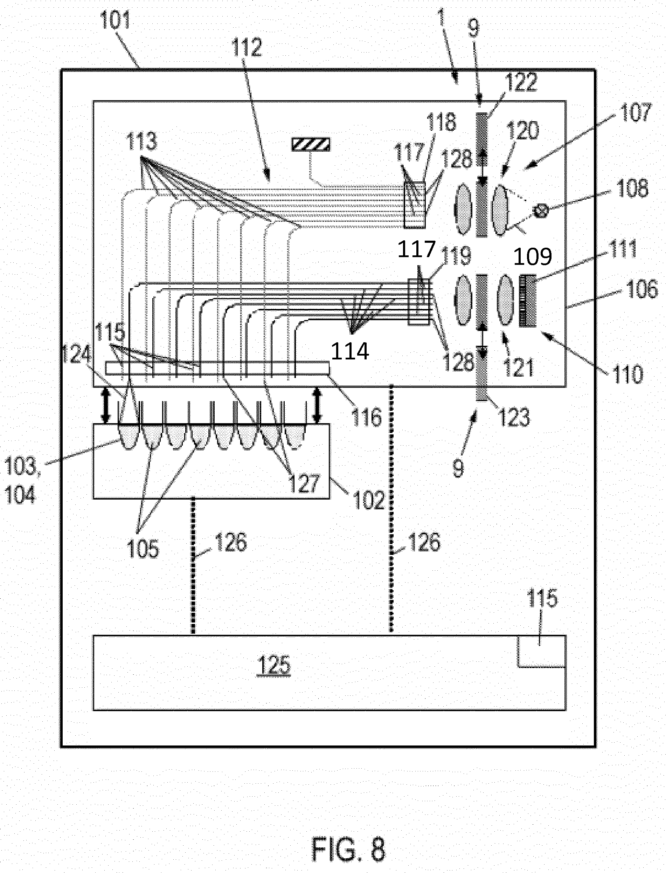

As illustrated in FIG. 5, the filter arrangement 9 of the instrument 1 can also be configured for translational movement. Stated more particularly, the filter arrangement 9 includes a filter unit 12' comprising two one-piece filter strips 14, i.e. a first filter strip 14 and a second filter strip 14, each of which having a non-continuous or discrete transmission spectrum. Stated more particularly, as illustrated in FIG. 7, each filter strip 14 includes a plurality of discrete filter portions 13 which are serially arranged with respect to each other. Specifically, each filter strip 14 includes a row of discrete filter portions 13 having transmission maxima, e.g., at 470, 490, 510, 530, 550, 570, 590, 610, 630, 650 and 670 nm. Relating to dyes having a Stokes shift of about 40 nm wherein the emission peak of one dye essentially corresponds to the excitation peak of another dye, a pair of first and second portions 13 related to one dye is formed by two distinct filter portions, the first filter portion 13 being provided by the first filter strip 14 and the second filter portion 13 being provided by the second filter strip 14.
With continued reference to FIG. 5, each filter strip 14 is operatively coupled to an individual drive 10 for translational movement. Accordingly, each filter strip 14 can be separately moved so that the first and second filter portions 13 of one pair of filter portions related to one dye can be moved in operative positions in which the first and second portions 13 are positioned in the excitation and emission beam paths 5, 8 while each other filter portion 13 is positioned outside the excitation and emission beam paths 5, 8.
The filter arrangement 9 of the instrument 1 can also include a filter unit 12" comprising a single filter strip 14 having a discrete transmission spectrum as illustrated in FIG. 6. Being operatively coupled to only one drive 10, different dyes preferably, but not limited to having an at least approximately equal Stokes shift wherein the emission peak of one dye essentially corresponds to the excitation peak of another dye can be excited and detected by moving the filter strip 14 by equal moving steps. Specifically, as illustrated, first and second filter portions 13 being separated by one filter portion can be related to one dye. Accordingly, cross-talk can effectively be prevented.
Now referring to FIG. 8, an exemplary system for the automated thermal treating of liquid samples generally referred to at reference numeral 101 comprising an instrument 1 as described above in connection with the FIGS. 1 through 7 is explained. The instrument 101 is configured as a thermo-cycler for thermally cycling reaction mixtures of nucleic acids and one or more reagents through a series of temperature excursions and optically detecting the reaction products obtained by means of fluorescence. The instrument 101 can, e.g., be used to perform the PCR, in particular real-time PCR, or any other reaction of the nucleic acid amplification type. Particularly, the instrument 101 can be used for the optical on-line detection of PCR reaction products and/or for the isothermal treatment or execution of melting curves.

Stated more particularly, the instrument 101 includes various modules which are functional and (optionally) structural entities for treating liquid samples. Specifically, the instrument 101 includes a thermal module 102 which can be brought in thermal communication with a multiwell plate 103 provided with plural cavities or wells 104 for receiving liquid samples 105. The thermal module 102 thus serves as a mount supporting the multiwell plate 103. The thermal module 102 can be heated or cooled according to pre-defined temperature profiles so as to transfer heat in controlled manner to/from the samples 105. A detection module 106 can be used to detect light so as to identify reaction products which, e.g., can be obtained as a result of a polymerase chain reaction of the samples 105, wherein the instrument 101 can be used for the optical on-line detection of the reaction products during progress of the amplification reactions. As indicated by the double arrows, the detection module 106 can at least vertically be moved in controlled manner relative to the thermal module 102, e.g., by means of a driven rack and pinion mechanism or any other mechanism enabling a vertical movement of the detection module 106. Accordingly, the detection module 106 can be moved in a lowered first position adapted for optically detecting reaction products obtained from the samples 105 or in a raised second or loading/unloading position adapted for loading or unloading the instrument 101 with the multi-well plate 103.

The detection module 106 includes an excitation arrangement 107 provided with at least one light source 108 for generating excitation light 109 adapted to excite the emission of light 124 (e.g. fluorescence light), in the following denoted as "emitted light", by the samples 105. As illustrated, the detection module 106 includes a detection arrangement 110 provided with at least one detector 111 to optically detect the emitted light 124. The detection module 106 further includes a coupling arrangement generally referred to at reference numeral 112 for optically coupling each of the excitation arrangement 107 and the detection arrangement 110 to the wells 104. Stated more particularly, the coupling arrangement 112 includes plural first optical fibers 113, in the following denoted as "excitation fibers", for transmitting the excitation light 109 from the excitation arrangement 107 to the wells 104 and plural second optical fibers 114, in the following denoted as "emission fibers", for transmitting the emitted light 124 from the wells 104 to the detection arrangement 110. Each well 104 of the multi-well plate 103 is related to an individual pair of one excitation fiber 113 and one emission fiber 114.

As further illustrated in FIG. 8, well-sided first end portions 115 of the excitation fibers 113 are fixed with respect to each other by means of a first fixing element 116, while second end portions 117 of the excitation fibers 113 opposite to the first end portions 115 thereof are fixed with respect to each other by a second fixing element 118. Otherwise, well-sided first end portions 115 of the emission fibers 114 are fixed with respect to each other by means of the first fixing element 116, while second end portions 117 of the emission fibers 114 opposite to the first end portions 115 thereof are fixed with respect to each other by a third fixing element 119. Specifically, the excitation light 109 can be coupled into the excitation fibers 113 at second end faces 128 and be coupled out of the excitation fibers 113 at first end faces 127 thereof. Otherwise, the emitted light 124 can be coupled into the emission fibers 114 at first end faces 127 and be coupled out of the emission fibers 114 at second end faces 128.
With continued reference to FIG. 8, an excitation optics generally referred to at reference numeral 120 is used to optically couple the excitation light 109 into the excitation fibers 113 at the second end faces 128. Specifically, one or more excitation filters 122 which are components of a filter arrangement 9 as above describe in connection with FIGS. 1 through 7 are used for filtering one or more specific wavelengths or one or more ranges of wavelengths before the excitation light 109 is coupled into the excitation fibers 113. In case the detection module 106 is in operative position, the first end faces 127 of the excitation fibers 113 are arranged in such a manner that the excitation light 109 is directed into the wells 104 to excite the emitted light 124 by the samples 105.
With continued reference to FIG. 8, in case the detection module 106 is in operative position, the first end faces 127 of the emission fibers 114 are arranged in such a manner that the emitted light 124 can be coupled into the emission fibers 114. An emission optics generally referred to at reference numeral 121 is used to optically couple the emitted light 124 leaving the emission fibers 114 at the second end faces 128 to the detector 111. One or more emission filters 123 which are components of a filter arrangement 9 as above describe in connection with FIGS. 1 through 7 are used for filtering one or more wavelengths or one or more ranges of wavelengths from the emitted light 124 before the emitted light 124 hits the detector 111.

A controller 125 is used for controlling the automated thermal treating of the samples 105. The controller 125 is configured as micro-controller running a computer-readable program provided with instructions to perform operations in accordance with a pre-defined sequence of steps. Specifically, the controller 125 receives information from the various components of the system 101, especially from the detector 111, and generates and transmits corresponding control signals to the components which require control. As schematically illustrated in FIG. 8, electric lines 126 are used for transmitting the electric signals.

### Reference list

- 1: Instrument
- 2: Analyte region
- 3: Excitation arrangement
- 4: Light source
- 5: Excitation beam path
- 6: Detection arrangement
- 7: Detector
- 8: Emission beam path
- 9: Filter arrangement
- 10: Drive
- 11: Controller
- 12, 12',12": Filter unit
- 13: Filter portion
- 14: Filter strip
- 15: Filter wheel
- 16: Wheel casing
- 17: Wheel hub
- 18: Base portion
- 19: Rim portion
- 20: Toothing
- 21: Pinion
- 22: Shaft
- 23: Motor
- 24: Opening
- 25: Seat
- 26: Spin axis
- 27: Sample
- 28: Fiber
- 29: Pair
- 30: Excitation light
- 31: Emitted light
- 32: Moving mechanism
- 33: Opaque region
- 34: Mount
- 35: Sample vessel
- 101: Instrument
- 102: Thermal module
- 103: Multiwell plate
- 104: Well
- 105: Liquid sample
- 106: Detection module
- 107: Excitation arrangement
- 108: Light source
- 109: Excitation light
- 110: Detection arrangement
- 111: Detector
- 112: Coupling arrangement
- 113: Excitation fiber
- 114: Emission fiber
- 115: First end portion
- 116: First fixing element
- 117: Second end portion
- 118: Second fixing element
- 119: Third fixing element
- 120: Excitation optics
- 121: Emission optics
- 122: Excitation filter
- 123: Emission filter
- 124: Emitted light
- 125: Controller
- 126: Electric line
- 127: First end face
- 128: Second end face

## Claims

1. An instrument (1) for detecting at least one analyte in a sample (27), comprising:
at least one light source (4), capable of generating excitation light (30) for exciting two or more luminescence labels,
an excitation beam path (5) extending between said light source (4) and said sample (27) for propagation of said excitation light (30),
at least one detector (7), capable of detecting light (31) emitted from said sample (27), an emission beam path (8) extending between said sample (27) and said detector (7) for propagation of said emitted light (31), and
at least one mount (34) for holding a sample carrier (35) or for accommodating a substrate with one or more analytes,
**characterized in that** the instrument (1) further comprises:
one filter carrier (14, 15) carrying more than two pairs (29) of filter portions (13), each pair (29) being related to one luminescence label and comprising a first filter portion (13), adapted to transmit excitation light (30) for exciting said related luminescence label and a second filter portion (13), adapted to transmit light (31) emitted by said related luminescence label, wherein said first filter portion (13) of at least one pair (29) is said second filter portion (13) of another pair (29), and wherein
said filter portions (13) are arranged in a manner that a respective one of said pairs (29) can be brought in an operative condition in which said first filter portion (13) is in said excitation beam path (5) and said second filter portion (13) is in said emission beam path (8), and wherein
said filter carrier (14, 15) and said beam paths (5, 8) are movable with respect to each other by at least one moving mechanism (32) so as to bring a respective one of said pairs (29) in said operative condition,
wherein said moving mechanism (32) is adapted to rotate or translate said filter carrier (15) in distinct positions with respect to said beam paths (5, 8) being stationary so as to rotate or translate a respective one of said pairs (29) of first and second filter portions (13) in operative condition and, wherein said filter portions (13) are arranged in a manner that a respective one of said pairs (29) of first and second filter portions (13) can be moved in operative condition by moving said filter carrier (14, 15) by equal moving steps with respect to said beam paths (5, 8) being stationary.

2. The instrument (1) according to claim 1, wherein said moving mechanism (32) comprises one carrier drive (10) for moving said filter carrier (14, 15) in distinct positions relative to said excitation and emission beam paths (5, 8).

3. The instrument (1) according to any one of the preceding claims 1 to 2, wherein said filter carrier (14, 15) includes at least one opaque region (33), adapted to inhibit the transmission of light (31) emitted by said sample, said opaque region (33) being arranged in a manner to be brought in said emission beam path (8).

4. The instrument (1) according to any one of the preceding claims 1 to 3, comprising a one-piece filter (14) for providing said first and second filter portions (13).

5. The instrument (1) according to claim 4, wherein said one-piece filter (14) has a discrete transmission spectrum.

6. A method for detecting at least one analyte in a sample, comprising the following steps of:
generating excitation light (30) propagating along an excitation beam path (5) towards said sample;
detecting light (31) emitted from said sample propagating along an emission beam path (8);
moving one pair (29) of first and second filter portions (13) related to one luminescence label and said beam paths (5, 8) with respect to each other so that said pair (29) is in an operative condition in which said first filter portion (13), adapted to transmit excitation light (30) for exciting said related luminescence label, is in said excitation beam path (5) and said second filter portion (13), adapted to transmit light (31) emitted by said related luminescence label, is in said emission beam path (8), moving another pair (29) of first and second filter portions (13) related to another luminescence label and said beam paths (5, 8) with respect to each other so that said another pair (29) is in said operative condition,
**characterized in that** the first filter portion (13) of the one pair (29) of filter portions (13) is the second filter portion (13) of the another pair (29) of filter portions, wherein a filter carrier (15) carrying said first and second filter portions (13) is rotated in distinct rotating positions or translated in distinct translating positions relative to said excitation and emission beam paths (5, 8) kept stationary so as to move a respective one of said pairs (29) of filter portions (13) in said operative condition, and
wherein said filter carrier (14, 15) is moved by equal moving steps.

7. A system (101) for analyzing a sample, comprising:
a instrument according to anyone of the preceding claims 1 to 5;
a temperature-controlled block (102) for heating said sample (105).

## Patentansprüche

1. Instrument (1) zum Detektieren mindestens eines Analyten in einer Probe (27), umfassend:
mindestens eine Lichtquelle (4), die fähig ist, Anregungslicht (30) zum Anregen von zwei oder mehr Lumineszenzmarkierungen zu erzeugen,
einen Anregungsstrahlweg (5), der sich zur Fortpflanzung des Anregungslichts (30) zwischen der Lichtquelle (4) und der Probe (27) erstreckt,
mindestens einen Detektor (7), der fähig ist, von der Probe (27) emittiertes Licht (31) zu detektieren, wobei sich ein Emissionsstrahlweg (8) zur Fortpflanzung des emittierten Lichts (31) zwischen der Probe (27) und dem Detektor (7) erstreckt, und mindestens eine Halterung (34) zum Halten eines Probenträgers (35) oder zum Unterbringen eines Substrats mit einem oder mehreren Analyten,
**dadurch gekennzeichnet, dass** das Instrument (1) ferner umfasst:
einen Filterträger (14, 15), der mehr als zwei Paare (29) von Filterabschnitten (13) trägt, wobei jedes Paar (29) zu einer Lumineszenzmarkierung gehört und einen ersten Filterabschnitt (13), der eingerichtet ist, um Anregungslicht (30) zum Anregen der zugehörigen Lumineszenzmarkierung zu übertragen, und einen zweiten Filterabschnitt (13), der eingerichtet ist, um von der zugehörigen Lumineszenzmarkierung emittiertes Licht (31) zu übertragen, umfasst, wobei der erste Filterabschnitt (13) mindestens eines Paares (29) der zweite Filterabschnitt (13) eines anderen Paares (29), ist und wobei
die Filterabschnitte (13) derart angeordnet sind, dass ein jeweiliges der Paare (29) in einen Betriebszustand gebracht werden kann, in dem sich der erste Filterabschnitt (13) im Anregungsstrahlweg (5) befindet und sich der zweite Filterabschnitt (13) im Emissionsstrahlweg (8) befindet, und wobei
der Filterträger (14, 15) und die Strahlwege (5, 8) in Bezug zueinander durch mindestens einen Bewegungsmechanismus (32) beweglich sind, um ein jeweiliges der Paare (29) in den Betriebszustand zu bringen,
wobei der Bewegungsmechanismus (32) eingerichtet ist, den Filterträger (15) in unterscheidbare Positionen bezüglich der stationären Strahlwege (5, 8) zu drehen oder zu verschieben, um so ein jeweiliges der Paare (29) aus erstem und zweitem Filterabschnitt (13) in den Betriebszustand zu drehen oder zu verschieben, wobei die Filterabschnitte (13) derart angeordnet sind, dass ein jeweiliges der Paare (29) aus erstem und zweitem Filterabschnitt (13) in den Betriebszustand bewegt werden kann, indem der Filterträger (14, 15) durch gleiche Bewegungsschritte bezüglich der stationären Strahlwege (5, 8) bewegt wird.

2. Instrument (1) nach Anspruch 1, wobei der Bewegungsmechanismus (32) einen Trägerantrieb (10) zur Bewegung des Filterträgers (14, 15) in unterscheidbare Positionen bezüglich des Anregungs- und des Emissionsstrahlwegs (5, 8) umfasst.

3. Instrument (1) nach einem der vorhergehenden Ansprüche 1 bis 2, wobei der Filterträger (14, 15) mindestens einen opaken Bereich (33) aufweist, der eingerichtet ist, die Übertragung von von der Probe emittiertem Licht (31) zu hemmen, wobei der opake Bereich (33) derart angeordnet ist, in den Emissionsstrahlweg (8) gebracht zu werden.

4. Instrument (1) nach einem der vorhergehenden Ansprüche 1 bis 3, umfassend einen einstückigen Filter (14) zum Bereitstellen des ersten und des zweiten Filterabschnitts (13).

5. Instrument (1) nach Anspruch 4, wobei der einstückige Filter (14) ein diskretes Übertragungsspektrum aufweist.

6. Verfahren zum Detektieren mindestens eines Analyten in einer Probe, umfassend die folgenden Schritte:
Erzeugen von Anregungslicht (30), das sich entlang eines Anregungsstrahlwegs (5) zur Probe hin fortpflanzt;
Detektieren von Licht (31), das von der Probe emittiert wird und sich entlang eines Emissionsstrahlwegs (8) fortpflanzt;
Bewegen eines Paars (29) aus erstem und zweitem Filterabschnitt (13), das zu einer Lumineszenzmarkierung gehört, und der Strahlwege (5,8) bezüglich zueinander, so dass sich das Paar (29) in einem Betriebszustand befindet, in dem sich der erste Filterabschnitt (13), der eingerichtet ist, Anregungslicht (30) zum Anregen der zugehörigen Lumineszenzmarkierung zu übertragen, im Anregungsstrahlweg (5) befindet und sich der zweite Filterabschnitt (13), der eingerichtet ist, von der zugehörigen Lumineszenzmarkierung emittiertes Licht (31) zu übertragen, im Emissionsstrahlweg (8) befindet, wobei ein anderes Paar (29) aus erstem und zweitem Filterabschnitt (13), das zu einer anderen Lumineszenzmarkierung gehört, und die Strahlwege (5,8) bezüglich zueinander bewegt werden, so dass sich das andere Paar (29) im Betriebszustand befindet,
**dadurch gekennzeichnet, dass** der erste Filterabschnitt (13) des einen Paars (29) von Filterabschnitten (13) der zweite Filterabschnitt (13) des anderen Paars (29) von Filterabschnitten ist, wobei ein Filterträger (15), der den ersten und den zweiten Filterabschnitt (13) trägt, bezüglich des stationär gehaltenen Anregungs- und
Emissionsstrahlwegs (5, 8) in unterscheidbare Drehpositionen gedreht oder in unterscheidbare Verschiebepositionen verschoben wird, um ein jeweiliges der Paare (29) von Filterabschnitten (13) in den Betriebszustand zu bewegen, und wobei der Filterträger (14, 15) durch gleiche Bewegungsschritte bewegt wird.

7. System (101) zum Analysieren einer Probe, umfassend:
ein Instrument nach einem der vorhergehenden Ansprüche 1 bis 5;
einen temperaturgesteuerten Block (102) zum Erwärmen der Probe (105).

## Revendications

1. Instrument (1) pour détecter au moins un analyte dans un échantillon (27), comprenant :
au moins une source de lumière (4), capable de générer une lumière d'excitation (30) pour exciter deux marqueurs de luminescence ou plus,
un trajet de faisceau d'excitation (5) s'étendant entre ladite source de lumière (4) et ledit échantillon (27) pour une propagation de ladite lumière d'excitation (30),
au moins un détecteur (7), capable de détecter une lumière (31) émise par ledit échantillon (27),
un trajet de faisceau d'émission (8) s'étendant entre ledit échantillon (27) et ledit détecteur (7) pour une propagation de ladite lumière (31) émise, et
au moins un support (34) pour contenir un porte-échantillon (35) ou pour loger un substrat avec un ou plusieurs analytes,
**caractérisé en ce que** l'instrument (1) comprend en outre :
un porte-filtre (14, 15) portant plus de deux paires (29) de portions de filtre (13), chaque paire (29) étant liée à un marqueur de luminescence et comprenant une première portion de filtre (13), adaptée pour transmettre une lumière d'excitation (30) pour exciter ledit marqueur de fluorescence lié et une seconde portion de filtre (13), adaptée pour transmettre une lumière (31) émise par ledit marqueur de luminescence lié, dans lequel ladite première portion de filtre (13) d'au moins une paire (29) est ladite seconde portion de filtre (13) d'une autre paire (29), et dans lequel
lesdites portions de filtre (13) sont agencées de manière à ce qu'une paire respective desdites paires (29) puisse être amenée dans un état opérationnel dans lequel ladite première portion de filtre (13) est dans ledit trajet de faisceau d'excitation (5) et ladite seconde portion de filtre (13) est dans ledit trajet de faisceau d'émission (8), et dans lequel ledit porte-filtre (14, 15) et lesdits trajets de faisceau (5, 8) sont mobiles les uns par rapport aux autres par au moins un mécanisme de déplacement (32) de façon à amener une paire respective desdites paires (29) dans ledit état opérationnel,
dans lequel ledit mécanisme de déplacement (32) est adapté pour mettre en rotation ou en translation ledit porte-filtre (15) dans des positions distinctes par rapport auxdits trajets de faisceau (5, 8) qui sont fixes de façon à mettre en rotation ou en translation une paire respective desdites paires (29) de première et seconde portions de filtre (13) en état opérationnel et, dans lequel lesdites portions de filtre (13) sont agencées de manière à ce qu'une paire respective desdites paires (29) de première et seconde portions de filtre (13) puisse être déplacée en état opérationnel en déplaçant ledit porte-filtre (14, 15) par des étapes de déplacement égales par rapport auxdits de trajets de faisceau (5, 8) qui sont fixes.

2. Instrument (1) selon la revendication 1, dans lequel ledit mécanisme de déplacement (32) comprend un mécanisme d'entraînement de porte-filtre (10) pour déplacer ledit porte-filtre (14, 15) dans des positions distinctes par rapport auxdits trajets de faisceau d'excitation et d'émission (5, 8).

3. Instrument (1) selon l'une quelconque des revendications 1 et 2 précédentes, dans lequel ledit porte-filtre (14, 15) comporte au moins une région opaque (33), adaptée pour entraver la transmission de lumière (31) émise par ledit échantillon, ladite région opaque (33) étant agencée de manière à être amenée dans ledit trajet de faisceau d'émission (8).

4. Instrument (1) selon l'une quelconque des revendications 1 à 3 précédentes, comprenant un filtre monobloc (14) pour fournir lesdites première et seconde portions de filtre (13).

5. Instrument (1) selon la revendication 4, dans lequel ledit filtre monobloc (14) a un spectre de transmission discret.

6. Procédé de détection d'au moins un analyte dans un échantillon, comprenant les étapes suivantes de :
génération d'une lumière d'excitation (30) se propageant le long d'un trajet de faisceau d'excitation (5) vers ledit échantillon ;
détection d'une lumière (31) émise par ledit échantillon se propageant le long d'un trajet de faisceau d'émission (8) ;
déplacement d'une paire (29) de première et seconde portions de filtre (13) liée à un marqueur de luminescence et desdits trajets de faisceau (5, 8) les uns par rapport aux autres de sorte que ladite paire (29) soit dans un état opérationnel dans lequel ladite première portion de filtre (13), adaptée pour transmettre une lumière d'excitation (30) pour exciter ledit marqueur de fluorescence lié, est dans ledit trajet de faisceau d'excitation (5) et ladite seconde portion de filtre (13), adaptée pour transmettre une lumière (31) émise par ledit marqueur de luminescence lié, est dans ledit trajet de faisceau d'émission (8), déplacement d'une autre paire (29) de première et seconde portions de filtre (13) liée à un autre marqueur de luminescence et desdits trajets de faisceau (5, 8) les uns par rapport aux autres de sorte que ladite autre paire (29) soit dans ledit état opérationnel,
**caractérisé en ce que** la première portion de filtre (13) de ladite paire (29) de portions de filtre (13) est la seconde portion de filtre (13) de l'autre paire (29) de portions de filtre, dans lequel un porte-filtre (15) portant lesdites première et seconde portions de filtre (13) est mis en rotation dans des positions de rotation distinctes ou mis en translation dans des positions de translation distinctes par rapport auxdits trajets de faisceau d'excitation et d'émission (5, 8) maintenus fixes de façon à déplacer une paire respective desdites paires (29) de portions de filtre (13) dans ledit état opérationnel, et
dans lequel ledit porte-filtre (14, 15) est déplacé par des étapes de déplacement égales.

7. Système (101) pour analyser un échantillon, comprenant :
un instrument selon l'une quelconque des revendications 1 à 5 précédentes ;
un bloc à régulation de température (102) pour chauffer ledit échantillon (105).
